# EUROPEAN PATENT APPLICATION

(11) **EP 4 837 568 A1**
(43) Date of publication of application: **11.11.2026**
(21) Application number: 24917309.7
(22) Date of filing: 04.12.2024
(51) Int. Cl.: A61K 9/51, A61K 47/69, A61K 51/12, A61K 51/08, A61P 35/00

(54) **SURFACE-MODIFIED ALBUMIN NANO-PLATFORM, METHOD FOR PREPARING SAME, AND COMPOSITION FOR DIAGNOSING OR TREATING DISEASE USING SAME**

(30) Priority: 11.01.2024 KR 20240004801
(71) Applicant: Seoul National University R&DB Foundation, Seoul 08826 (KR)
(72) Inventor: LEE, Yun Sang, Yongin-si Gyeonggi-do 16851 (KR); CHUNG, Yujin, Yongin-si Gyeonggi-do 16817 (KR); SEOK, Seung Hyeok, Seoul 04153 (KR); CHUNG, Hyewon, Seoul 06253 (KR); PARK, Jisu, Seoul 08050 (KR); PARK, Ji Yong, Incheon 22002 (KR); YOO, Ran Ji, Seoul 01727 (KR); KIM, Kyuwan, Seoul 02264 (KR); KIM, Ji Yoon, Seoul 05587 (KR); KIM, Jin Sil, Seoul 02863 (KR)
(74) Representative: AWA Sweden AB
(86) International application number: PCT/KR2024/019735
(87) International publication number: WO 2025/150713

(57) **Abstract**

The present invention relates to a surface-modified albumin nanoplatform, a method for preparing the same, and a composition for diagnosing or treating a disease using the same. The surface-modified albumin nanoplatform according to the present invention can reduce uptake by the reticuloendothelial system even when an excessive amount of click functional groups and various transmitters are conjugated to albumin, by deactivating the click-reactive functional groups on the surface of the albumin to which a transmitter is not conjugated, and accordingly, the in vivo half-life is greatly increased, so that a target molecule or active material can be effectively delivered to a target tissue or cell, thereby achieving excellent therapeutic and diagnostic effects.

## Description

### [Technical Field]

The present invention relates to a surface-modified albumin nanoplatform, a method for preparing the same, and a composition for diagnosing or treating a disease using the same, and more particularly, to an albumin nanoplatform having an increased in vivo half-life and a method for preparing the same, in which a click functional group on a surface of the albumin nanoplatform to which a transmitter is not conjugated is masked and thereby deactivated to reduce uptake by the reticuloendothelial system, and to a composition for diagnosing or treating a disease using the same.

### [Background Art]

Albumin is a highly stable protein that circulates in the blood for 20 days or more, and is used as a drug delivery system that improves the in vivo stability of various drugs by chemically conjugating a conventional low-molecular-weight chemical drug, protein, antibody, or the like to albumin. Such a drug delivery system using albumin has been applied to diagnosis and treatment and utilized in various pharmaceutical fields. Representatively, research has been conducted in which a hydrophobic anticancer agent such as doxorubicin or methotrexate (MTX) is chemically conjugated to albumin to improve cancer-targeting ability and to improve the in-blood stability of the drug.

In addition, research on preparing nanoparticles using albumin has also been actively conducted. Since albumin is a biodegradable material that is non-toxic, non-immunogenic, and degradable in the body, nanoparticles formed of albumin are very useful as drug carriers.

However, with respect to nanoparticle-based drug delivery methods using various materials such as albumin, liposomes, or antibodies, many studies have confirmed that a nanoparticle-based carrier, when injected into the body, is rapidly taken up by the reticuloendothelial system, including the liver or the spleen, and thus exhibits a short in vivo drug residence time, which has limited its utilization. To solve this problem, there is a method of increasing the amount of the nanoparticle-based drug carrier used; however, there is a toxicity problem depending on the physical-property composition of the nanoparticles, problems regarding immune responses have always been raised, and even up to now, the development of technology for solving the short in vivo drug residence time of nanoparticles is insufficient.

Korean Patent Registration No. 10-1043407 discloses a technology in which hydrophilic albumin and a hydrophobic bile acid are conjugated to form a complex for drug delivery; however, the protein complex forms a self-assembly in a hydrophilic environment to deliver a drug in the form of nanoparticles, is particularly directed to hydrophobic drug delivery, and does not disclose an effect of increasing the in vivo half-life.

As described above, since the importance of albumin nanoparticles as drug carriers is drawing attention, there is a need to develop a technology for delaying the uptake of albumin nanoparticles by the reticuloendothelial system to increase the in vivo drug residence time.

### [Disclosure]

### [Technical Problem]

An object of the present invention is to provide a surface-modified albumin nanoplatform for increasing the in vivo half-life.

Another object of the present invention is to provide a method for preparing a surface-modified albumin nanoplatform having an increased in vivo half-life.

Still another object of the present invention is to provide a composition for diagnosing or treating a disease, comprising a surface-modified albumin nanoplatform having an increased in vivo half-life.

### [Technical Solution]

The present invention provides a surface-modified albumin nanoplatform obtained by a click chemistry reaction between albumin conjugated with an azide (N₃) or cyclooctyne functional group and a transmitter conjugated with an azide (N₃) or cyclooctyne functional group, wherein when the albumin is conjugated with the azide functional group, the transmitter is conjugated with the cyclooctyne functional group, and when the albumin is conjugated with the cyclooctyne functional group, the transmitter is conjugated with the azide functional group, and wherein at least one of the azide or cyclooctyne functional groups conjugated to the albumin, to which the transmitter is not conjugated, is inactivated.

In the present invention, the azide or cyclooctyne functional group introduced into the albumin may range from 1 to 30.

In the present invention, the inactivation may be performed by conjugating a masking material having a substituent selected from the group consisting of an amino group (-NH₂), a carboxyl group (-COOH), and a hydroxyl group (-OH) to the azide or cyclooctyne functional group conjugated to the albumin.

In the present invention, the conjugation of the masking material may be performed by subjecting a masking material conjugated with an azide or cyclooctyne functional group to a click chemistry reaction with the albumin conjugated with the transmitter.

In the present invention, when the albumin is conjugated with the azide functional group, the masking material may be conjugated with the cyclooctyne functional group, and when the albumin is conjugated with the cyclooctyne functional group, the masking material may be conjugated with the azide functional group.

In the present invention, the transmitter may be at least one selected from the group consisting of a saccharide compound, folic acid or a derivative thereof, RGD (arginyl-glycyl-aspartic acid) or a derivative thereof, an active material, a radioactive isotope, and a fluorescent material.

In the present invention, the saccharide compound may be at least one selected from the group consisting of a glucosyl group, a mannosyl group, and a galactosyl group.

In the present invention, the radioactive isotope may be at least one selected from the group consisting of ³H, ¹¹C, ¹⁸F, ¹⁴Cl, ³²P, ³⁵S, ³⁶Cl, ⁴⁵Ca, ⁵¹Cr, ⁵⁷Co, ⁵⁸Co, ⁵⁹F, ⁶⁴Cu, ⁶⁷Ga, ⁶⁸Ga, ⁸⁹Zr, ⁹⁰Y, ⁹⁹Mo, ^{99m}Tc, ¹¹¹In, ¹³¹I, ¹²⁵I, ¹²⁴I, ¹²³I, ¹⁸⁶Re, ¹⁸⁸Re, ²²⁵Ac, ²¹²Pb, ^{117m}Sn, and ¹⁷⁷Lu.

In the present invention, the radioactive isotope may be labeled by a chelating agent, and the chelating agent may be at least one selected from the group consisting of NOTA (1,4,7-triazacyclononane-1,4,7-triacetic acid), DOTA (1,4,7,10-tetraazacyclododecane-1,4,7,10-tetraacetic acid), DFO (3-[6,17-dihydroxy-7,10,18,21-tetraoxo-27-[N-acetylhydroxylamino]-6,11,17,22-tetraazaheptaeicosane]thiourea), DTPA (diethylenetriaminepentaacetic acid), N₂S₂ (diaminedithiol), p-SCN-Bn-NOTA (2-(4'-isothiocyanatobenzyl)-1,4,7-triazacyclononane-1,4,7-triacetic acid), NODAGA (1,4,7-triazacyclononane,1-glutaric acid-4,7-acetic acid), p-SCN-Bn-DOTA (2-(4'-isothiocyanatobenzyl)-1,4,7,10-tetraazacyclododecane-1,4,7,10-tetraacetic acid), TETA (1,4,8,11-tetraazacyclotetradecane-1,4,8,11-tetraacetic acid), p-SCN-Bn-DTPA (2-(4-isothiocyanatobenzyl)-diethylenetriaminepentaacetic acid), p-SCN-Bn-DFO (1-(4-isothiocyanatophenyl)-3-[6,17-dihydroxy-7,10,18,21-tetraoxo-27-[N-acetylhydroxylamino)-6,11,17,22-tetraazaheptaeicosane]thiourea), and HYNIC (hydrazinonicotinic acid).

In the present invention, the fluorescent material may be at least one selected from the group consisting of FNR (Ferrodoxin NADP(+) reductase), a cyanine-based fluorescent material, TAMRA (tetramethylrhodamine-5-maleimide), a Flamma^{®} fluorescent material, and ICG (indocyanine green).

The present invention also provides a method for preparing a surface-modified albumin nanoplatform, comprising: (1) preparing a solution of albumin conjugated with an azide (N₃) or cyclooctyne functional group; (2) mixing the solution prepared in step (1) with a solution comprising a transmitter conjugated with a cyclooctyne functional group or an azide (N₃) functional group, and subjecting the mixture to a click chemistry reaction without a copper catalyst (Cu-free click chemistry); and (3) mixing the albumin solution conjugated with the transmitter with a solution comprising a masking material conjugated with a cyclooctyne functional group or an azide (N₃) functional group, and subjecting the mixture to a click chemistry reaction without a copper catalyst (Cu-free click chemistry), wherein when the albumin is conjugated with the azide functional group, the transmitter and the masking material are conjugated with the cyclooctyne functional group, and when the albumin is conjugated with the cyclooctyne functional group, the transmitter and the masking material are conjugated with the azide functional group.

In the method of the present invention, in step (3), a molar ratio of the albumin to the masking material may be 1:10 to 1:100.

The present invention also provides a composition for diagnosing or treating a disease, comprising the surface-modified albumin nanoplatform.

In the present invention, the disease may be selected from acute nephritis, periodontitis, sarcopenia, pancreatic cancer, head and neck cancer, endometrial cancer, bone cancer, breast cancer, central or peripheral nervous system cancer, gastrointestinal cancer, germ cell cancer, adenocarcinoma, hematologic cancer, renal-urinary tract cancer, liver cancer, lung cancer, pleural cancer, prostate cancer, sarcoma, and skin cancer.

### [Advantageous Effects]

The surface-modified albumin nanoplatform according to the present invention can reduce uptake by the reticuloendothelial system even when an excessive amount of click functional groups and various transmitters are conjugated to albumin, by deactivating the click-reactive functional groups on the surface of the albumin to which a transmitter is not conjugated, and accordingly, the in vivo half-life is greatly increased, so that a target molecule or active material can be effectively delivered to a target tissue or cell, thereby achieving excellent therapeutic and diagnostic effects.

### [Description of Drawings]

FIG. 1 shows the results of confirming the residual amount in the blood in the body by confirming the fluorescence expression level of non-masking and masking albumin, according to an embodiment of the present invention.
FIG. 2 shows PET image results of confirming the fluorescence expression in the blood, liver tissue, and cancer tissue in vivo after administering non-masked mannosylated albumin (⁶⁴Cu-Alb-Man) and masked mannosylated albumin (⁶⁴Cu-Alb-Man-blocking) to a tumor animal model, according to an embodiment of the present invention.
FIG. 3A is a graph showing the expression level in the blood among the PET images of FIG. 2, according to an embodiment of the present invention.
FIG. 3B is a graph showing the fluorescence expression level in the liver tissue among the PET images of FIG. 2, according to an embodiment of the present invention.
FIG. 4 is a graph showing the fluorescence expression level in the tumor among the PET images of FIG. 2, according to an embodiment of the present invention.

### [Best Mode for Invention]

Hereinafter, specific embodiments of the present invention will be described in more detail. Unless defined otherwise, all technical and scientific terms used herein have the same meaning as commonly understood by those skilled in the art to which the present invention belongs. In general, the nomenclature used herein is well known and commonly used in the art.

Throughout the specification, when a part is said to "comprise" a certain component, this means that it may further include other components rather than excluding other components, unless specifically stated to the contrary.

The present invention provides a surface-modified albumin-based nanoplatform for increasing the in vivo half-life and a method for preparing the same.

The present inventors have previously proposed, in a prior study, an albumin nanoplatform having a clicked surface functional group by reacting with an amine functional group on the surface of albumin (Patent No. 10-1879682, Albumin-based disease-tracking diagnostic or therapeutic nanoplatform). The prior study proposed that albumin and a click functional group react at a molar ratio of 1:1 to 1:12, because in a reaction using a higher molar ratio, there was a problem in that the utilization of the nanoplatform was greatly limited due to rapid uptake by the reticuloendothelial system.

However, in order to introduce a functional group clicked with various target materials or drugs, it is more effective to react the click reactive groups at a molar ratio of 1:12 or more to introduce as many as nine or more clicked functional groups onto the surface of albumin.

Therefore, the present invention is intended to provide an albumin nanoplatform in which a large number of click-reactive functional groups are conjugated to the surface of albumin while reducing the uptake rate in the liver and greatly improving the in vivo half-life.

Specifically, in the present invention, in order to increase the in vivo half-life of an albumin nanoplatform having a click-reactive functional group conjugated to its surface, a masking material is conjugated to the click-reactive functional groups on the surface of the albumin nanoplatform to which a transmitter is not conjugated to deactivate the same, thereby reducing uptake by the reticuloendothelial system, so as to provide an albumin nanoplatform having a greatly increased in vivo half-life.

In this aspect, the present invention provides an albumin nanoplatform obtained by a click chemistry reaction between albumin conjugated with an azide (N₃) or cyclooctyne functional group and a transmitter conjugated with an azide (N₃) or cyclooctyne functional group, wherein when the albumin is conjugated with the azide functional group, the transmitter is conjugated with the cyclooctyne functional group, and when the albumin is conjugated with the cyclooctyne functional group, the transmitter is conjugated with the azide functional group, wherein at least one of the azide or cyclooctyne functional groups of the albumin nanoplatform, to which the transmitter is not conjugated, is inactivated.

Albumin is one of the proteins constituting the basic substance of cells, is present in a very large amount in the blood, and refers to a protein produced in the liver. The albumin has the smallest molecular weight among simple proteins present in a natural state. Serum albumin in the blood has a function of maintaining and restoring the plasma volume, and thus is used to prevent shock due to excessive bleeding and in surgery and burn treatment, and is also known to have an oxygen-delivering ability similar to that of hemoglobin.

The albumin may include any albumin that can be formulated into a preparation, but may preferably be one derived from human plasma or a recombinant human serum albumin prepared by genetic engineering, without being limited thereto. Genetic information on the albumin of the present invention can be obtained from a known database such as NCBI GenBank.

The number of amino groups (-NH₂) exposed on the surface of the albumin may be 15 to 35.

In a specific embodiment of the present invention, in order to prepare a nanoplatform that can exhibit an effect of treating or diagnosing a disease by delivering a transmitter such as a drug or a fluorescent material by targeting a target tissue in vivo based on biocompatible albumin, representative human serum albumin (HSA) was used, and a cyclooctyne or azide (N₃) group, which is one of the functional groups of click chemistry, was introduced onto the surface of the human serum albumin under reaction conditions that minimize denaturation of the HSA.

In the present invention, the azide (N₃) is a reactive group consisting of three nitrogen atoms, has high reactivity, and is known to play a role of an electron donor in a 1,3-dipolar cycloaddition reaction, which is a type of Cu-Free click chemistry, to form a triaza-5-membered ring.

The cyclooctyne functional group is an 8-membered aliphatic ring comprising a triple bond under ring strain, and is known to play a role of an electron acceptor particularly in a 1,3-dipolar cycloaddition reaction, which is a type of Cu-Free click chemistry, to form a triaza-5-membered ring. Due to the structural feature of cyclooctyne, that is, the triple-bond structure under ring strain, click chemistry is enabled even without a Cu(I) catalyst.

The cyclooctyne functional group may be at least one selected from the group consisting of 4-cyclooctyn-1-yl ( 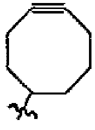 ), 3-cyclooctyn-1-yl ( 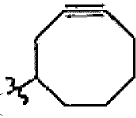 ), 2-cyclooctyn-1-yl ( 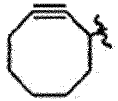 ), Monofluorinated cyclooctyne (MOFO) ( 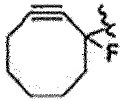 ), Difluororinated cyclooctyne (DIFO) ( 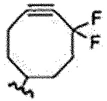 ), Dimethoxyazacyclooctyne (DIMAC) ( 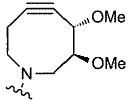 ), Dibenzocyclooctyne (DIBO) ( 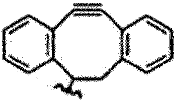 ), Azadibenzocyclooctyne (ADIBO) group ( 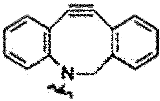 ), and biarylazacyclooctynone (BARAC) ( 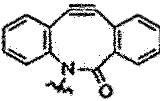 ), but is not necessarily limited thereto.

The albumin conjugated with the azide (N₃) or cyclooctyne functional group can be obtained by: (a) dissolving albumin in phosphate-buffered saline (PBS); (b) dissolving azide-NHS or cyclooctyne-NHS in DMSO; and (c) mixing the obtained solutions and then reacting them at 20 to 37°C for 30 minutes to 1 hour.

In step (a), the phosphate-buffered saline (PBS) may have a pH of 6.8 to 7.6, preferably a pH of 7.0 to 7.4.

In step (b), the amount of DMSO used to prepare the azide-NHS solution or cyclooctyne-NHS solution may be 2% (v/v) or less of the total reaction solution.

In step (c), the mixing molar ratio of the albumin to the azide-NHS or cyclooctyne-NHS may be 1:1 to 1:30.

When the albumin solution and the azide-NHS solution are mixed in step (c), the functional group conjugated to the albumin may be an azide functional group, and when the albumin solution and the cyclooctyne-NHS solution are mixed in step (c), the functional group conjugated to the albumin may be a cyclooctyne functional group.

In an embodiment of the present invention, a human serum albumin (HSA) solution and an ADIBO-NHS solution were mixed and reacted at 37°C for 30 minutes to prepare HSA-ADIBO.

In the present invention, the number of click-reactive functional groups (azide or cyclooctyne functional groups) introduced onto the surface of albumin by such a method is preferably 1 to 30, and more preferably 9 to 20.

In the prior art of the present inventors, the number of click-reactive functional groups was limited because, when the number of click-reactive functional groups exceeds 10, they are taken up by the liver immediately upon injection into the body, so that uptake to another target disease site may be limited. However, according to the present invention, since liver uptake is limited by inactivating the click-reactive functional groups to which a transmitter is not conjugated, a larger number of click-reactive functional groups can be introduced, so that more various transmitters can be conjugated, and at the same time, there is an effect that the residence period in the body is prolonged.

The number of click-reactive functional groups introduced onto the surface of albumin can be adjusted according to the reaction ratio of the albumin to the azide-NHS or cyclooctyne-NHS.

In the present invention, a masking material can be reacted with the click-reactive functional groups conjugated to the surface of albumin to inactivate the same. As the masking material, a material capable of inactivating the click-reactive functional group can be used, and specifically, it is a material having a substituent selected from the group consisting of an amino group (-NH₂), a carboxyl group (-COOH), and a hydroxyl group (-OH). In particular, in terms of allowing the click-reactive functional group to be recognized as the surface of albumin, a material having an amino group is most preferable. As the compound comprising the amino group and carboxyl group, for example, lysine can be used, and as the compound comprising the hydroxyl group, for example, polyethylene glycol (PEG) can be used.

The masking material reacts with the click-reactive functional groups that are not conjugated with a transmitter on the surface of the albumin nanoplatform to deactivate the same, and returns the albumin nanoplatform to an albumin surface state having the original amino or carboxyl functional groups, so that, without affecting the targeting or therapeutic ability of the transmitter, uptake by the reticuloendothelial system is reduced, whereby the in vivo half-life of the albumin nanoplatform can be greatly increased.

In an embodiment of the present invention, as a result of confirming the in vivo distribution of albumin depending on whether masking was performed, in the mice injected with the non-masking albumin nanoplatform, uptake into the liver appeared rapidly as the DOF increased, whereas in the mice administered with the albumin nanoplatform masked with an amine group, the fluorescence level was maintained for a long time even at a high DOF, confirming that the albumin nanoplatform surface-modified with the masking material had reduced liver uptake and an increased residual amount in the blood, so that the circulation half-life was increased.

In the present invention, the albumin nanoplatform is obtained by mixing the solution comprising the albumin conjugated with the azide or cyclooctyne functional group described above with a solution comprising a transmitter conjugated with an azide or cyclooctyne functional group and subjecting the mixture to a click chemistry reaction, wherein the click chemistry reaction may be a copper-catalyst-free (Cu-free) click chemistry reaction.

In one aspect of the present invention, since the azide functional group used as the click chemistry functional group is an electron donor and the cyclooctyne functional group is an electron acceptor, when the functional group conjugated to the albumin is an azide functional group, the functional group conjugated to the transmitter is preferably a cyclooctyne functional group, and when the functional group conjugated to the albumin is a cyclooctyne functional group, the functional group conjugated to the transmitter is preferably an azide functional group.

Next, the solution of the albumin nanoplatform conjugated with the transmitter is mixed with a solution comprising a masking material conjugated with an azide or cyclooctyne functional group and subjected to a click chemistry reaction, whereby a surface-modified albumin nanoplatform can be obtained. In this case, the azide or cyclooctyne functional group conjugated to the masking material is preferably the same as the functional group conjugated to the transmitter.

For example, as the azide-conjugated masking material, H-L-Lys(N₃)-OH, H-L-Aha-OH, H-L-Lys(N₃)-OH*HCl, H-L-Aha-OH*HCl, H-L-Phe(4-N₃)-OH, 3-azido-1-propanol, 3-azidopropionic acid, or the like can be used, and the compound may comprise PEG as a linker.

In this aspect, the surface-modified albumin nanoplatform of the present invention can be prepared by: (1) preparing a solution of albumin conjugated with an azide (N₃) or cyclooctyne functional group; (2) mixing the solution prepared in step (1) with a solution comprising a transmitter conjugated with a cyclooctyne functional group or an azide (N₃) functional group, and subjecting the mixture to a click chemistry reaction without a copper catalyst (Cu-free click chemistry); and (3) mixing the albumin solution conjugated with the transmitter with a solution comprising a masking material conjugated with a cyclooctyne functional group or an azide (N₃) functional group, and subjecting the mixture to a click chemistry reaction without a copper catalyst (Cu-free click chemistry).

In this case, when a solution in which an azide (N₃) group is conjugated to an albumin monomer is used in step (1), it is preferable to react with a solution comprising a transmitter and a masking material conjugated with a cyclooctyne group in steps (2) and (3), and when a solution in which a cyclooctyne functional group is conjugated to an albumin monomer is used in step (1), it is preferable to react with a solution comprising a transmitter and a masking material conjugated with an azide (N₃) functional group in steps (2) and (3).

In step (3), the albumin conjugated with the transmitter and the masking material may be mixed at a molar ratio of 1:10 to 1:100, preferably 1:20 to 1:60. This can be adjusted according to the number of functional groups, among the click-reactive functional groups conjugated to the surface of albumin, to which a transmitter is not conjugated, and it is preferable to use an excessive amount of the masking material in the sense of inactivating as many click-reactive functional groups as possible. For example, the molar ratio of the albumin to the masking material may be 1:30 to 1:50. When the albumin and the masking material are reacted within the molar ratio range, all of the click-reactive functional groups to which a transmitter is not conjugated on the surface of albumin can be inactivated at a rapid rate of 2 to 6 hours.

The reaction of step (3) may be performed under a pH condition of 6 to 7 in order to maintain the stability of albumin. When the pH is higher than the range, thermal denaturation of the albumin may occur, and when the pH is lower than the range, decomposition and oxidative damage due to acidification may occur.

In addition, the reaction temperature of step (3) may be 0 to 60°C, but is preferably performed at room temperature in order to induce a more rapid conjugation reaction between the click-reactive functional group and the masking material while maintaining the stability of the albumin.

Since the albumin nanoplatform of the present invention has a large number of click-reactive functional groups, various transmitters can be conjugated thereto. The binding material that can be conjugated to the albumin nanoplatform of the present invention may be a single material or may be a combination of various binding materials.

When the transmitter conjugated to the albumin nanoplatform is a combination of two or more materials, the combination of two or more materials may be conjugated to the albumin nanoplatform simultaneously, or each material may be conjugated sequentially, without being limited thereto.

In the present invention, the transmitter refers to a material that is conjugated to the albumin nanoplatform and delivered in vivo, and may be selected from the group consisting of a target molecule, an active material, a radioactive isotope, a fluorescent material, and a combination thereof.

The term "target molecule" of the present invention refers to a molecule that targets a specific environment in vivo, and specifically refers to a molecule that targets a material present in a specific environment in vivo to guide the albumin nanoplatform of the present invention to the corresponding specific environment. The target molecule of the present invention can guide the albumin nanoplatform of the present invention to a specific environment, or can confirm the presence or absence of a specific environment, the location of a specific environment, the degree of a specific environment, and the like. In the present invention, the specific environment in vivo targeted by the target molecule may be a tissue-specific specific environment or a disease-specific specific environment. That is, the target molecule may target a material present tissue-specifically or a material present disease-specifically. The material present tissue-specifically may be a protein, RNA, accumulated or generated compound, or the like that is present tissue-specifically, and the material present disease-specifically may be a protein, RNA, accumulated or generated compound, or the like that is present depending on the type of disease.

In particular, in the present invention, the target molecule may be a saccharide compound, folate or a derivative thereof that targets a folate receptor present in large numbers in cancer tissue, or RGD (arginyl-glycyl-aspartic acid) or a derivative thereof.

In an embodiment of the present invention, the transmitter may be a saccharide compound, and the saccharide compound may be a mannosyl group, a galactosyl group, or a glucosyl group. The structures of the mannosyl group, the galactosyl group, and the glucosyl group are as shown in the following Formulae 1 to 3, respectively.

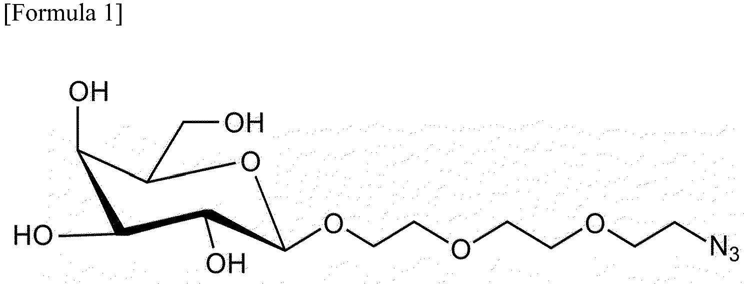

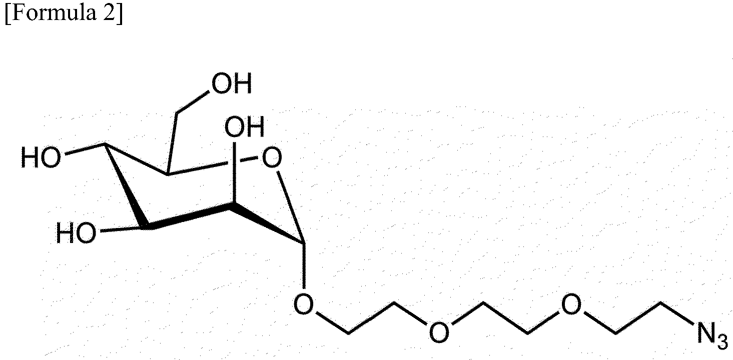

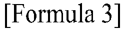

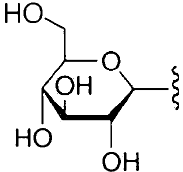

Due to such a saccharide compound transmitter, the albumin nanoplatform, which is one aspect of the present invention, has a targeting ability for macrophages, which has been disclosed in detail in the prior study of the present inventors, Patent No. 10-2366189.

Specifically, an albumin nanoplatform comprising a mannosyl group or a galactosyl group as a transmitter has a targeting ability for macrophages, whereby drug delivery to macrophages becomes possible, and as a result, it can function as a platform for preemptive diagnosis and treatment of metastatic cancer.

The mannose receptor is known to be widely distributed in cells responsible for defense mechanisms, among which a representative immune cell is the Kupffer cell. When the transmitter is a mannosyl group, since it targets Kupffer cells, it mostly acts in the liver, but action may also occur in the blood, muscle, spleen, and lung.

In addition, when the transmitter is a galactosyl group, it may exhibit hepatobiliary excretion through the gall bladder. Furthermore, the albumin-based nanoplatform of the present invention conjugated with a glucosyl group as a transmitter has a targeting ability for M1-type macrophages, whereby it is selectively taken up by inflammatory macrophages in damaged tissue, suppresses the inflammatory response and ROS generation induced by inflammatory macrophages to restore the function of damaged mitochondria, and suppresses cell death of damaged tissue, so that it can be used as a nanoplatform capable of preventing or treating the progression to a serious disease.

In the present invention, the albumin nanoplatform may comprise 4 to 8 saccharide compounds, and more preferably 5 to 7.

In the present invention, folic acid may be conjugated as the transmitter.

Folic acid is a type of vitamin, also called vitamin B9 or vitamin M. Folic acid forms a specific binding with a folate receptor. The folate receptor is a tumor-associated glycosylphosphatidylinositol-anchored protein, and allows conjugated folic acid or a folic-acid-conjugated material to be taken up through receptor-mediated endocytosis.

In the present invention, RGD as the transmitter is a tripeptide consisting of three amino acids, namely arginine (L-arginine), glycine, and aspartic acid (L-aspartic acid), and was first discovered at the cell adhesion site of fibronectin. Since they have a strong affinity for the integrin receptor mainly expressed in the endothelial cell layer of tumor blood vessels, they are widely used as a universal means in research such as drug-targeted therapy and simple reagents.

The term "active material" of the present invention may be a natural product, vitamin, active agent, biologic, compound, amino acid, protein, peptide, nucleotide, nucleic acid, polypeptide, polynucleotide, and analogs, homologs, and derivatives thereof, and in particular may be selected from an anti-obesity drug, a central nervous system stimulant, a carotenoid, a corticosteroid, an elastase inhibitor, an antifungal agent, a tumor therapeutic agent, an antiemetic, an analgesic, a cardiovascular agent, an anti-inflammatory agent, an anthelmintic, an antiarrhythmic agent, an antibiotic, an anticoagulant, an antidepressant, an antidiabetic agent, an antiepileptic, an antihistamine, an antihypertensive agent, an antimuscarinic agent, an antimycobacterial agent, an immunosuppressant, an antithyroid agent, an antiviral agent, an anxiolytic, a sedative, an astringent, an alpha-adrenergic receptor blocker, a beta-adrenergic receptor blocker, a blood preparation, a cardiotonic agent, a contrast medium, a cough suppressant, a diagnostic reagent, a diagnostic imaging agent, a diuretic, a dopamine agonist, a hemostatic agent, an immunological agent, a lipid-regulating agent, a muscle relaxant, a parasympathomimetic agent, an antiallergic agent, an appetite suppressant, a sympathomimetic agent, a thyroid agent, a vasodilator, and a combination thereof.

In the present invention, the radioactive isotope as the transmitter may be labeled by a chelating agent.

A radioactive isotope refers to elements having the same atomic number but different atomic weights, and among isotopes, those having radioactivity are referred to as radioactive isotopes. Such a radioactive isotope can be used as an important marker for diagnosing a disease or confirming pharmacokinetics by utilizing the property of attenuating radioactivity by emitting gamma rays or other subatomic particles.

The radioactive isotope that can be used as a marker in the present invention can be used without limitation as long as it is known in the art, and may be at least one selected from the group consisting of ³H, ¹¹C, ¹⁸F, ¹⁴Cl, ³²P, ³⁵S, ³⁶Cl, ⁴⁵Ca, ⁵¹Cr, ⁵⁷Co, ⁵⁸Co, ⁵⁹F, ⁶⁴Cu, ⁶⁷Ga, ⁶⁸Ga, ⁸⁹Zr, ⁹⁰Y, ⁹⁹Mo, ^{99m}Tc, ¹¹¹In, ¹³¹I, ¹²⁵I, ¹²⁴I, ¹²³I, ¹⁸⁶Re, ¹⁸⁸Re, ²²⁵Ac, ²¹²Pb, ^{117m}Sn, and ¹⁷⁷Lu, and preferably ¹¹C, ¹⁸F, ⁶⁴Cu, ⁶⁷Ga, ⁶⁸Ga, ⁸⁹Zr, ^{99m}Tc, ¹¹¹In, and ¹²³I, but is not necessarily limited thereto.

The chelating agent serves to connect the radioactive isotope to albumin, and may be, for example, at least one selected from the group consisting of NOTA (1,4,7-triazacyclononane-1,4,7-triacetic acid), DOTA (1,4,7,10-tetraazacyclododecane-1,4,7,10-tetraacetic acid), DFO (3-[6,17-dihydroxy-7,10,18,21-tetraoxo-27-[N-acetylhydroxylamino]-6,11,17,22-tetraazaheptaeicosane]thiourea), DTPA (diethylenetriaminepentaacetic acid), N₂S₂ (diaminedithiol), p-SCN-Bn-NOTA (2-(4'-isothiocyanatobenzyl)-1,4,7-triazacyclononane-1,4,7-triacetic acid), NODAGA (1,4,7-triazacyclononane,1-glutaric acid-4,7-acetic acid), p-SCN-Bn-DOTA (2-(4'-isothiocyanatobenzyl)-1,4,7,10-tetraazacyclododecane-1,4,7,10-tetraacetic acid), TETA (1,4,8,11-tetraazacyclotetradecane-1,4,8,11-tetraacetic acid), p-SCN-Bn-DTPA (2-(4-isothiocyanatobenzyl)-diethylenetriaminepentaacetic acid), p-SCN-Bn-DFO (1-(4-isothiocyanatophenyl)-3-[6,17-dihydroxy-7,10,18,21-tetraoxo-27-[N-acetylhydroxylamino)-6,11,17,22-tetraazaheptaeicosane]thiourea), and HYNIC (hydrazinonicotinic acid), but is not necessarily limited thereto.

In the present invention, the transmitter may further comprise a fluorescent material.

A fluorescent material refers to a material that emits a specific wavelength of visible light with respect to a specific wavelength, and in the present invention, includes all fluorescent materials that can be conjugated to the albumin-based macrophage-targeting nanoplatform and used to confirm the location of the nanoplatform.

Specifically, the fluorescent material that can be used as a marker in the present invention can be used without limitation as long as it is known in the art, and a fluorescent material such as a rhodamine-based material including rhodamine, TAMRA, and the like; a fluorescein-based material including fluorescein, FITC (fluorescein isothiocyanate), and FAM (fluorescein amidite); a bodipy (boron-dipyrromethene); an alexa fluor-based material; and a cyanine-based material including Cy3, Cy5, Cy7, and indocyanine green can be used, without being limited thereto.

The surface-modified albumin nanoplatform according to the present invention has a long in vivo half-life, and thus can be utilized as a composition for diagnosing or treating a disease by maintaining a target molecule and/or an active material in the body for a long period of time.

The disease is not limited as long as it is any disease that can be diagnosed or treated by the transmitter conjugated to the albumin nanoplatform of the present invention, and may be, for example, an inflammatory disease such as acute nephritis, periodontitis, or sarcopenia, or a cancer such as pancreatic cancer, head and neck cancer, endometrial cancer, bone cancer, breast cancer, central/peripheral nervous system cancer, gastrointestinal cancer, germ cell cancer, adenocarcinoma, hematologic cancer, renal-urinary tract cancer, liver cancer, lung/pleural cancer, prostate cancer, sarcoma, or skin cancer.

As described above, the albumin nanoplatform prepared by the method of the present invention can reduce uptake by the reticuloendothelial system even when an excessive amount of click functional groups and various transmitters are conjugated to albumin, by deactivating the click functional groups on the surface of the albumin to which a transmitter is not conjugated, and accordingly, through the increased in vivo half-life, a target molecule or active material can be effectively delivered to a target tissue or cell, thereby achieving excellent therapeutic and diagnostic effects.

### Examples

Hereinafter, the present invention will be described in more detail through Examples. However, these Examples show some experimental methods and configurations in order to illustrate the present invention by way of example, and the scope of the present invention is not limited to these Examples.

### Preparation Example 1: Preparation of albumin-based nanoplatform

### 1-1. Preparation of albumin into which ADIBO functional group is introduced

Human serum albumin (HSA) was dissolved in PBS at a concentration of 5 mg/0.5 mL, 0.5 mL of the albumin was dispensed into an Eppendorf (EP) tube, and then ADIBO-NHS dissolved in DMSO was added at molar ratios of 1:3, 1:6, 1:12, 1:13, 1:16, 1:20, and 1:24 and rapidly dispersed by vortexing. Thereafter, after ultrasonication for 1 minute, the mixture was stirred and reacted at room temperature for 4 hours, and purified using a 30 kDa centrifugal filter.

The purified albumin was transferred in 0.5 mL portions to a new EP tube, and then the number of ADIBO functional groups introduced into the HSA-ADIBO was quantified by a UV-Vis spectrophotometric method (A280, A309) and by MALDI-TOF, and is shown in Table 1.

**[Table 1]**

| No. | Albumin (mg) | ADIBO-NHS (µL) | Albumin:ADIBO (molar ratio) | ADIBO DOF (MALDI-TOF) | Corrected A280 (A_{280c}) | DOF (UV-Vis) |
|---|---|---|---|---|---|---|
| 1 | 5 | - | 1:0 | - | 0.055 | - |
| 2 | 5 | 0.7 | 1:3 | 1.9 | 0.043 | 2.7 |
| 3 | 5 | 1.4 | 1:6 | 4.9 | 0.048 | 5.1 |
| 4 | 5 | 2.7 | 1:12 | 9.7 | 0.047 | 10.0 |
| 5 | 5 | 3 | 1:13 | 10.4 | 0.053 | 10.7 |
| 6 | 5 | 3.6 | 1:16 | 12.4 | 0.048 | 13.2 |
| 7 | 5 | 4.6 | 1:20 | 14.1 | 0.047 | 14.7 |
| 8 | 5 | 5.5 | 1:24 | 16.4 | 0.051 | 16.6 |

As a result of mixing at a maximum molar ratio of 1:24, it was confirmed that about 16 ADIBO can be conjugated.

### 1-2. Masking of albumin nanoplatform

Among the albumin nanoplatforms prepared as in Preparation Example 1-1, sample Nos. 2, 4, 6, and 8 (ADIBO numbers 2, 10, 12, and 16) were labeled with ⁶⁴Cu and then masked with N₃⁻NH₂.

First, in order to label ⁶⁴Cu on the ADIBO functional group on the surface of albumin, a vial containing ⁶⁴Cu was dried in a hood using N₂ gas for 30 minutes. Subsequently, 200 µL of 1 M sodium acetate buffer (pH 5) was added to the vial to adjust the pH to 5, and NOTA-N₃ (1 mg/mL in DW, 10 µL) was added and heated at 70°C for 5 minutes.

In order to measure the radiolabeling efficiency, thin-layer chromatography (TLC) was performed using ITLC-SG paper (Radio-TLC, with 0.1 M citric acid as the mobile phase). After confirming the radiolabeling efficiency, 10 µL (9 nmole, 0.5 mCi) of the radiolabeled NOTA-N₃ solution ([⁶⁴Cu]Cu-NOTA-N₃, 10 mCi/200 µL) was added to the Alb-ADIBO.

Alb was added to PBS (500 µg/0.5 mL, 15 nmole/0.5 mL) and reacted at room temperature (RT) for 30 minutes, and finally the Rf value of unlabeled ⁶⁴Cu, [⁶⁴Cu], was confirmed. As a result of performing radio-TLC to confirm the generation of radiolabeled Alb-ADIBO, it was confirmed that there was no unlabeled ⁶⁴Cu.

Next, 50 µL of 3-azido-1-propanamine dissolved in distilled water at a concentration of 100 mg/88 µL was added to the ⁶⁴Cu-labeled Alb-ADIBO (2 mg/0.8 mL), followed by blocking at 4°C and centrifugation at 10000 rpm at 4°C for 3 minutes to obtain masked albumin. The masking material was reacted at a molar ratio of about 40 times relative to the albumin, and based on the 16 ADIBO functional groups conjugated to the albumin, it was confirmed that ADIBO:N₃-NH₂ reacted at 1:25 or more.

### Experimental Example 1: Confirmation of in vivo distribution of albumin depending on masking

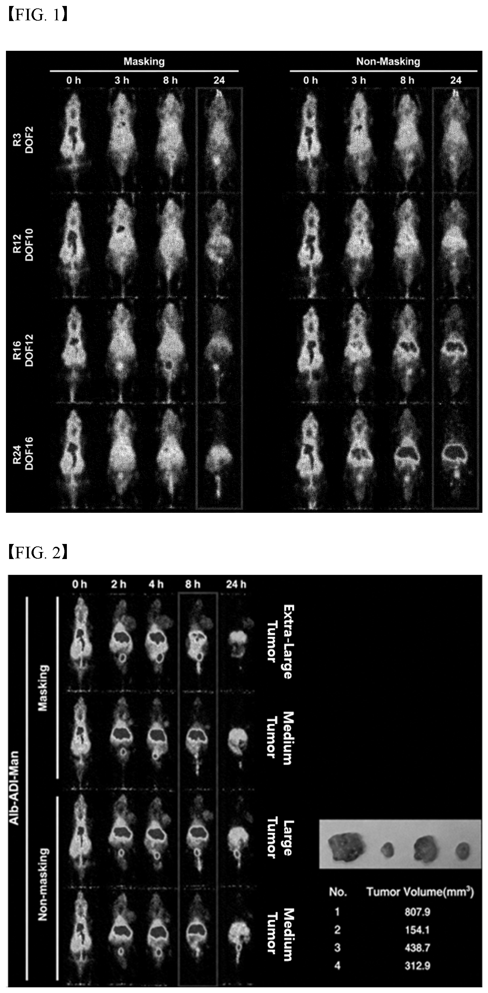

After masking the isotope-labeled albumin nanoplatform as in Preparation Example 1, it was administered to the tail vein of a normal mouse, and the fluorescence distribution was confirmed by time period (0, 3, 8, and 24 hours) using a PET (positron emission tomography) device, and is shown in FIG. 1.

Referring to FIG. 1, in the mouse injected with the non-masking albumin nanoplatform, it could be confirmed that uptake into the liver occurred rapidly as the DOF increased.

On the other hand, in the mouse administered with the albumin nanoplatform masked with N₃-NH₂, it was confirmed that a fluorescence level similar to that of DOF 10 appeared even at DOF 12 and DOF 16, which are higher than DOF 10.

Through this, it could be confirmed that the albumin nanoplatform masked with N₃-NH₂ had reduced liver uptake and an increased residual amount in the blood, so that the blood circulation half-life was greatly increased.

### Preparation Example 2: Preparation of masked glycosylated albumin nanoplatform

The HSA-ADIBO prepared as in Preparation Example 1-1 was dissolved in PBS, mixed with Man-N₃ (1-O-(2-(2-(2-azidoethoxy)ethoxy)ethoxy)-alpha-D-mannopyranoside) at an HSA-ADIBO:Man-N₃ molar ratio of 1:8, reacted at 37°C for 1 hour, and purified with a centrifugal filter tube to prepare albumin conjugated with a mannosyl group (Alb-Man).

Thereafter, by the method of Preparation Example 1-2, the albumin conjugated with a mannosyl group was labeled with ⁶⁴Cu and masked with N₃-NH₂ to obtain a surface-modified mannosylated albumin nanoplatform (⁶⁴Cu-Alb-Man-blocking).

### Experimental Example 2: Confirmation of residual amount of masked albumin nanoplatform in blood

The residual amount in the blood and the liver uptake level of the masked albumin nanoplatform were confirmed.

The surface-modified albumin nanoplatform (⁶⁴Cu-Alb-Man-Blocking) prepared by the method of Preparation Example 2 and a non-masked albumin nanoplatform control (⁶⁴Cu-Alb-Man) were each administered to the tail vein of a tumor mouse model. Thereafter, the fluorescence expression level by the isotope was confirmed through PET images of the tumor animal model, and is shown in FIG. 2.

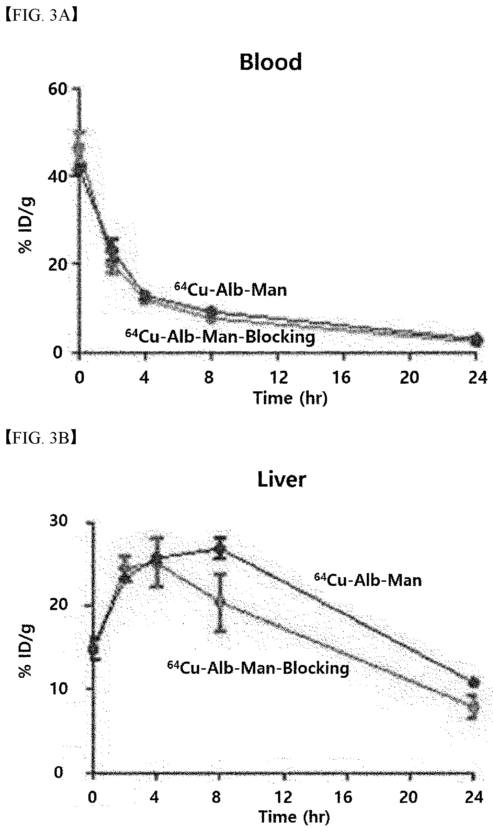

Referring to FIG. 3A, a graph showing the fluorescence expression level in the blood from the PET images of FIG. 2, and FIG. 3B, a graph showing the fluorescence expression level in the liver tissue, the residual amounts in the blood of the masked albumin nanoplatform and the non-masking control showed similar levels, but in the case of the non-masking control, it was confirmed that the residual amount in the blood decreased rapidly due to liver uptake. On the other hand, it was confirmed that the time for the masked albumin nanoplatform to be taken up by the liver was delayed.

### Experimental Example 3: Confirmation of targeting ability of masked glycosylated albumin nanoplatform

Through the PET images of the tumor animal models to which the masked mannosylated albumin nanoplatform (⁶⁴Cu-Alb-Man-Blocking) or the non-masking control (⁶⁴Cu-Alb-Man) of Experimental Example 2 was each administered, the isotope fluorescence expression level in the tumor tissue was confirmed to determine whether masking affects the tumor-targeting ability of the albumin nanoplatform.

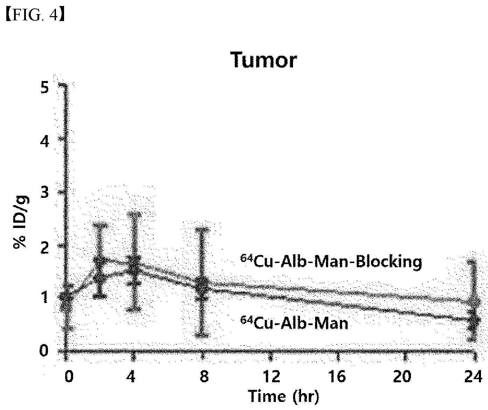

Referring to FIG. 4, which confirms the targeting ability of the non-masking or masking mannosylated albumin, it was confirmed that the masked albumin nanoplatform targeted the tumor at a level similar to that of the non-masking control, and through this, it could be confirmed that whether masking is performed does not affect the tumor-targeting ability of the albumin nanoplatform.

Taking the above results together, the masking method of the present invention, which modifies the surface of an albumin nanoplatform into which an excessive amount of ADIBO groups have been introduced, can improve the rapid liver uptake rate of the albumin nanoplatform to increase the in vivo circulation half-life, thereby increasing the disease-targeting rate to enhance the usefulness as a diagnostic agent, and can reduce the effect on liver toxicity during drug delivery.

Although some embodiments of the present invention have been described above, the present invention is not limited only to the embodiments described above, but can be modified and varied within a scope that does not depart from the gist of the present invention, and it should be understood that such modified and varied forms also belong to the technical spirit of the present invention.

## Claims

1. A surface-modified albumin nanoplatform obtained by a click chemistry reaction between albumin conjugated with an azide (N₃) or cyclooctyne functional group and a transmitter conjugated with an azide (N₃) or cyclooctyne functional group,
wherein when the albumin is conjugated with the azide functional group, the transmitter is conjugated with the cyclooctyne functional group, and when the albumin is conjugated with the cyclooctyne functional group, the transmitter is conjugated with the azide functional group, and
wherein at least one of the azide or cyclooctyne functional groups conjugated to the albumin, to which the transmitter is not conjugated, is inactivated.

2. The surface-modified albumin nanoplatform of claim 1,
wherein the azide or cyclooctyne functional group introduced into the albumin ranges from 1 to 30.

3. The surface-modified albumin nanoplatform of claim 1,
wherein the inactivation is performed by conjugating a masking material having a substituent selected from the group consisting of an amino group (-NH₂), a carboxyl group (-COOH), and a hydroxyl group (-OH) to the azide or cyclooctyne functional group conjugated to the albumin.

4. The surface-modified albumin nanoplatform of claim 3,
wherein the masking material is lysine or polyethylene glycol (PEG).

5. The surface-modified albumin nanoplatform of claim 3,
wherein the conjugation of the masking material is performed by subjecting a masking material conjugated with an azide or cyclooctyne functional group to a click chemistry reaction with the albumin conjugated with the transmitter.

6. The surface-modified albumin nanoplatform of claim 5,
wherein when the albumin is conjugated with the azide functional group, the masking material is conjugated with the cyclooctyne functional group, and when the albumin is conjugated with the cyclooctyne functional group, the masking material is conjugated with the azide functional group.

7. The surface-modified albumin nanoplatform of claim 1,
wherein the transmitter is at least one selected from the group consisting of a saccharide compound, folic acid or a derivative thereof, RGD (arginyl-glycyl-aspartic acid) or a derivative thereof, an active material, a radioactive isotope, and a fluorescent material.

8. The surface-modified albumin nanoplatform of claim 7,
wherein the saccharide compound is at least one selected from the group consisting of a glucosyl group, a mannosyl group, and a galactosyl group.

9. The surface-modified albumin nanoplatform of claim 7,
wherein the radioactive isotope is at least one selected from the group consisting of ³H, ¹¹C, ¹⁸F, ¹⁴Cl, ³²P, ³⁵S, ³⁶Cl, ⁴⁵Ca, ⁵¹Cr, ⁵⁷Co, ⁵⁸Co, ⁵⁹F, ⁶⁴Cu, ⁶⁷Ga, ⁶⁸Ga, ⁸⁹Zr, ⁹⁰Y, ⁹⁹Mo, ^{99m}Tc, ¹¹¹In, ¹³¹I, ¹²⁵I, ¹²⁴I, ¹²³I, ¹⁸⁶Re, ¹⁸⁸Re, ²²⁵Ac, ²¹²Pb, ^{117m}Sn, and ¹⁷⁷Lu.

10. The surface-modified albumin nanoplatform of claim 9,
wherein the radioactive isotope is labeled by a chelating agent, and the chelating agent is at least one selected from the group consisting of NOTA (1,4,7-triazacyclononane-1,4,7-triacetic acid), DOTA (1,4,7,10-tetraazacyclododecane-1,4,7,10-tetraacetic acid), DFO (3-[6,17-dihydroxy-7,10,18,21-tetraoxo-27-[N-acetylhydroxylamino]-6,11,17,22-tetraazaheptaeicosane]thiourea), DTPA (diethylenetriaminepentaacetic acid), N₂S₂ (diaminedithiol), p-SCN-Bn-NOTA (2-(4'-isothiocyanatobenzyl)-1,4,7-triazacyclononane-1,4,7-triacetic acid), NODAGA (1,4,7-triazacyclononane,1-glutaric acid-4,7-acetic acid), p-SCN-Bn-DOTA (2-(4'-isothiocyanatobenzyl)-1,4,7,10-tetraazacyclododecane-1,4,7,10-tetraacetic acid), TETA (1,4,8,11-tetraazacyclotetradecane-1,4,8,11-tetraacetic acid), p-SCN-Bn-DTPA (2-(4-isothiocyanatobenzyl)-diethylenetriaminepentaacetic acid), p-SCN-Bn-DFO (1-(4-isothiocyanatophenyl)-3-[6,17-dihydroxy-7,10,18,21-tetraoxo-27-[N-acetylhydroxylamino)-6,11,17,22-tetraazaheptaeicosane]thiourea), and HYNIC (hydrazinonicotinic acid).

11. The surface-modified albumin nanoplatform of claim 1,
wherein the fluorescent material is at least one selected from the group consisting of FNR (Ferrodoxin NADP(+) reductase), a cyanine-based fluorescent material, TAMRA (tetramethylrhodamine-5-maleimide), a Flamma^{®} fluorescent material, and ICG (indocyanine green).

12. A method for preparing a surface-modified albumin nanoplatform, comprising:
(1) a step of preparing a solution of albumin conjugated with an azide (N₃) or cyclooctyne functional group;
(2) a step of mixing the solution prepared in step (1) with a solution comprising a transmitter conjugated with a cyclooctyne functional group or an azide (N₃) functional group, and subjecting the mixture to a click chemistry reaction without a copper catalyst (Cu-free click chemistry); and
(3) a step of mixing an albumin solution conjugated with the transmitter with a solution comprising a masking material conjugated with a cyclooctyne functional group or an azide (N₃) functional group, and subjecting the mixture to a click chemistry reaction without a copper catalyst (Cu-free click chemistry),
wherein when the albumin is conjugated with the azide functional group, the transmitter and the masking material are conjugated with the cyclooctyne functional group, and when the albumin is conjugated with the cyclooctyne functional group, the transmitter and the masking material are conjugated with the azide functional group.

13. The method for preparing a surface-modified albumin nanoplatform of claim 12,
wherein in step (3), a molar ratio of the albumin to the masking material is 1:10 to 1:100.

14. A composition for diagnosing or treating a disease, comprising the surface-modified albumin nanoplatform of any one of claims 1 to 11.

15. The composition for diagnosing or treating a disease of claim 14,
wherein the disease is selected from acute nephritis, periodontitis, sarcopenia, pancreatic cancer, head and neck cancer, endometrial cancer, bone cancer, breast cancer, central or peripheral nervous system cancer, gastrointestinal cancer, germ cell cancer, adenocarcinoma, hematologic cancer, renal-urinary tract cancer, liver cancer, lung cancer, pleural cancer, prostate cancer, sarcoma, and skin cancer.
